# EUROPEAN PATENT APPLICATION

(11) **EP 4 349 988 A1**
(43) Date of publication of application: **10.04.2024**
(21) Application number: 22811338.7
(22) Date of filing: 24.05.2022
(51) Int. Cl.: C12N 15/52, C12N 1/21, C12N 9/00, C12N 15/74

(54) **METHOD FOR PRODUCING PLASMID, AND PLASMID**

(30) Priority: 25.05.2021 JP 2021087711
(71) Applicant: Spiber Inc., Tsuruoka-shi, Yamagata 997-0052 (JP)
(72) Inventor: LIPS, David, Tsuruoka-shi, Yamagata 997-0052 (JP); VERKLEIJ, Gijs, Tsuruoka-shi, Yamagata 997-0052 (JP)
(74) Representative: Handley, Matthew Edward
(86) International application number: PCT/JP2022/021313
(87) International publication number: WO 2022/250068

(57) **Abstract**

Provided are a method for producing a novel plasmid in heterologous expression of a gene and a plasmid. The method includes preparing a first gene cluster containing the plurality of genes by a DNA assembly method using a plasmid transformation system of Bacillus subtilis; and linking, to the first gene cluster, a second gene cluster containing a replication origin of Bacillus subtilis, a replication origin of Escherichia coli, and an initiation sequence for conjugation to an actinomycete.

## Description

### Technical Field

The present invention relates to a method for producing a plasmid using a DNA assembly method using a plasmid transformation system of Bacillus subtilis and a plasmid.

### Background Art

Natural compounds produced by microorganisms such as actinomycetes and filamentous fungi are known as useful substances having wide variety of structures and biological activities. In the present day, biosynthetic gene clusters can be easily identified by decoding genome of producing bacteria. It has also become clear that there are many gene clusters of useful substances that have not been used by humans. Among secondary metabolites of microorganisms, a gene cluster for biosynthesis of industrially important polyketide-based compounds and peptide-based compounds has been mainly studied. Examples thereof include a type I polyketide synthase (PKS), which is a type of multi-modular biosynthetic enzyme used for biosynthesis of macrolide compounds such as erythromycin, FK-506 (tacrolimus), rapamycin, and avermectin which are clinically applied secondary metabolites produced by actinomycetes.

For example, in consideration of the difficulty of producing a polyketide compound or the like by a traditional chemical method and typically low production of polyketides in wild-type cells, there is considerable interest in finding improved or alternative means for producing a polyketide compound. For these reasons, heterologous production of the compounds has been attempted by introducing a gene cluster required for the biosynthesis from the original strain into other cells.

In addition, for heterologous expression of a gene cluster, for example, a method of cloning an existing cluster from a genomic DNA sample, a method of de novo assembly from a synthetic DNA fragment, and the like are known. On the other hand, for the heterologous expression, since it is required to express genes (including gene clusters) between a plurality of hosts, it is required to modify a plasmid constructed by a previous host cell each time the host cell is changed, and a shuttle vector is known to solve the problem.

However, a plasmid required to be constructed has a problem that compatibility between host cells and compatibility by a synthesis method exist, and in heterologous expression of a gene, a novel method is still required.

### Citation List

### Patent Literature

Patent Literature 1: US 2010/0,291,633 A
Patent Literature 2: US 7,723,077
Patent Literature 3: JP 2011-512140 A

### Non Patent Literature

Non Patent Literature 1: PLoS One, 2009, 4(5), e5553
Non Patent Literature 2: Kagaku To Seibutsu, 2016, Vol. 54, No. 10, pp. 74

### Summary of Invention

### Technical Problem

An object of the present invention is to provide a method for producing a novel plasmid in heterologous expression of a gene and a plasmid.

### Solution to Problem

The present inventors have found that it is not required to prepare a first gene cluster containing a plurality of genes using a DNA assembly method using a plasmid transformation system of Bacillus subtilis, and to modify a plasmid for each host cell using a plasmid in which a second gene cluster containing a replication origin of Bacillus subtilis, a replication origin of Escherichia coli, and an initiation sequence for conjugation to an actinomycete and the first gene cluster are linked. The present invention is based on this novel finding.

The present invention provides, for example, the following inventions.
[1] A method for producing a plasmid containing a plurality of genes encoding a multi-modular biosynthetic enzyme, the method including: preparing a first gene cluster containing the plurality of genes by a DNA assembly method using Bacillus subtilis; and linking, to the first gene cluster, a second gene cluster containing a replication origin of Bacillus subtilis, a replication origin of Escherichia coli, and an initiation sequence for conjugation to an actinomycete.
[2] The method for producing a plasmid according to [1], in which the DNA assembly method using Bacillus subtilis is an OGAB method.
[3] A plasmid produced by the method according to [1] or [2] .
[4] An actinomycete containing a plasmid produced by the method according to [1] or [2].
[5] A method for producing a multi-modular biosynthetic enzyme, the method including producing a multi-modular biosynthetic enzyme in a host cell containing a plasmid produced by the method according to [1] or [2].
[6] The method for producing a multi-modular biosynthetic enzyme according to [5], in which the host cell is an actinomycete.
[7] The method for producing a multi-modular biosynthetic enzyme according to [6], in which the actinomycete is an actinomycete containing a plasmid obtained by conjugal transfer with Escherichia coli.
[8] The method for producing a multi-modular biosynthetic enzyme according to any one of [5] to [7], in which the multi-modular biosynthetic enzyme is a type I polyketide synthase (PKS).
[9] A multi-modular biosynthetic enzyme produced by the method according to any one of [5] to [8].
[10] A plasmid containing at least all of the following:
   (a) a replication origin of Bacillus subtilis; and
   (b) a replication origin of Escherichia coli.
[11] A DNA encoding a PKS having a homology of 80% or more with a DNA sequence encoding a PKS set forth in SEQ ID NO: 2.
[12] A plasmid having a homology of 80% or more with a DNA sequence related to a plasmid set forth in SEQ ID NO: 23.

### Advantageous Effects of Invention

The method for producing a plasmid of the present invention has an advantage that a plasmid in which a first gene cluster containing a plurality of genes encoding a multi-modular biosynthetic enzyme, and a second gene cluster containing a replication origin of Bacillus subtilis, a replication origin of Escherichia coli, and an initiation sequence for conjugation to an actinomycete, are linked, is used, such that there is no need to modify a plasmid for each host cell in order to perform expression in a heterologous expression host cell.

### Brief Description of Drawings

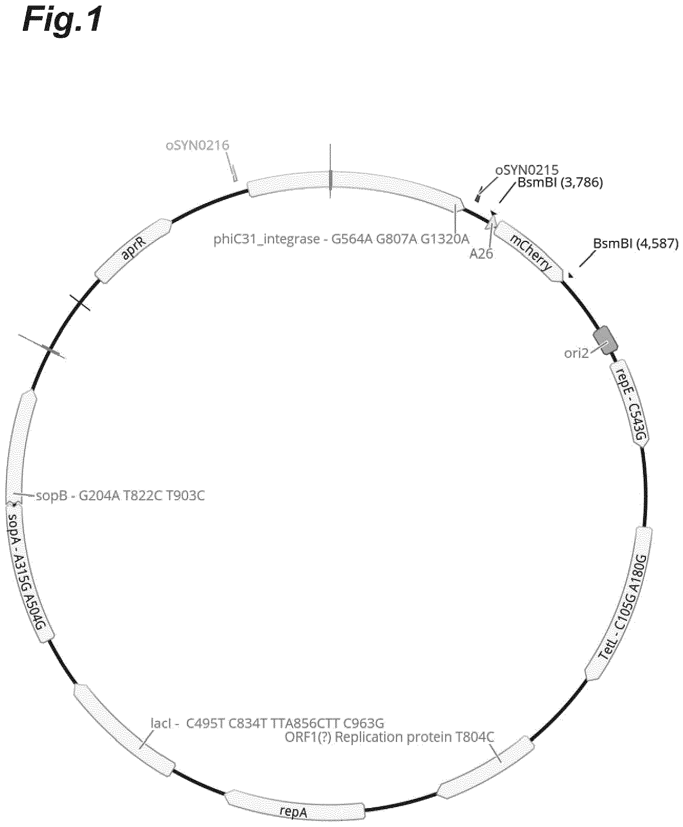

Fig. 1 is an explanatory diagram showing a plasmid map.

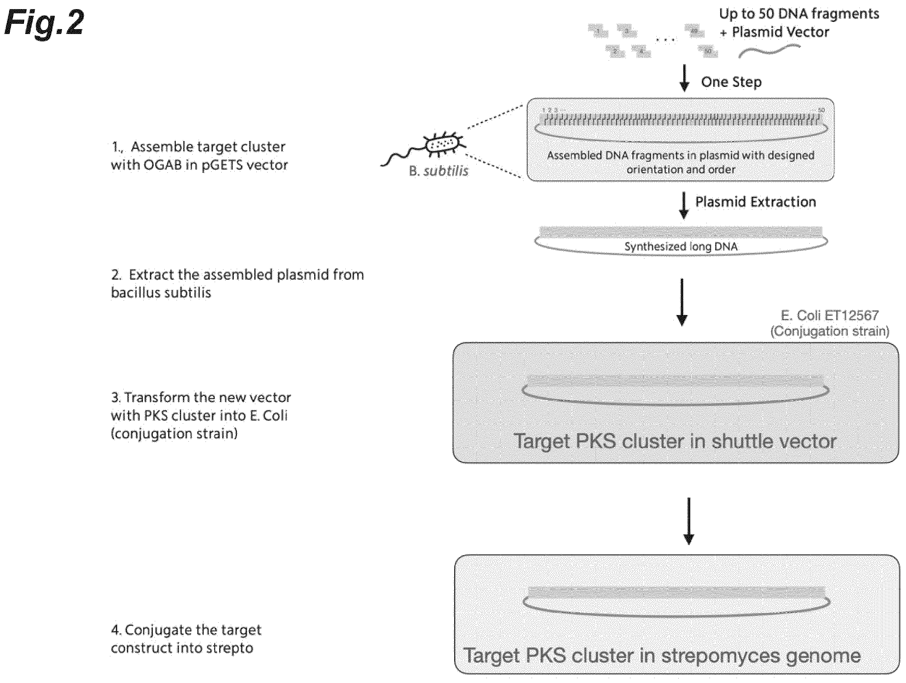

Fig. 2 is an explanatory diagram showing a method for producing a plasmid and a flow of gene expression in a heterologous expression host cell.

### Description of Embodiments

Hereinafter, embodiments of the present invention will be described in detail. However, the present invention is not limited to the following embodiments.

### [Multi-modular biosynthetic enzyme]

In the present specification, examples of a multi-modular biosynthetic enzyme include a type I polyketide synthase (PKS) and a nonribosomal peptide synthetase.

A DNA encoding a PKS or a nonribosomal peptide synthetase contained in the plasmid of the present invention may be a naturally occurring DNA, a DNA of which codon usage is modified, or a DNA of which one or two or more amino acids are modified. In one preferred embodiment, a Streptomyces PKS contains products of three open reading frames (ORF1, ORF2, and ORF3). A PKS contains three domains: a ketosynthase (KS) domain, an acyltransferase (AT) domain, and an acyl carrier protein (ACP). A polyketide chain can be elongated by these three domains. The PKS may further contain a domain associated with modification of the main chain, such as a ketoreductase (KR) domain, a dehydratase (DH) domain, or an enoyl reductase (ER) domain. Examples of a compound prepared by the PKS include 6-deoxyerythronolide B (6-dEB), frenolicin, granaticin, tetracenomycin, 6-methylsalicylic acid, oxytetracycline, tetracycline, erythromycin, griseusin, nanaomycin, medermycin, daunorubicin, tyrosine, carbomycin, spiramycin, avermectin, monensin, nonactin, curamycin, lipomycin, rifamycin, and candicidin.

### (PKS)

The type I polyketide synthase (PKS) is not particularly limited, and examples thereof include a PKS encoding a DNA sequence set forth in SEQ ID NO: 2, and may have a homology of 80% or more, 85% or more, 90% or more, 93% or more, 95% or more, 97% or more, or 99% or more with SEQ ID NO: 2.

Nonribosomal peptides are not particular limited, and refer to, for example, a class of peptides belonging to a family of complex natural products composed of simple amino acid monomers. The nonribosomal peptides are synthesized in many bacteria or fungi by large multifunctional proteins referred to as nonribosomal peptide synthetases (NRPSs). A feature of the NRPSs is the ability to synthesize a peptide containing proteinogenic and non-proteinogenic amino acids.

The "nonribosomal peptide synthetase" (NRPS) is not particularly limited, and refers to, for example, a large multifunctional protein organized in a cooperative group of active sites referred to as modules. Here, each module is required for catalyzing one cycle of peptide elongation and functional group modification. The number and order of the modules and the types of domains present in the modules on each NRPS instruct the number, order, and selection of the amino acids to be incorporated, as well as the modification associated with a specific type of elongation, thereby determining a structural variation in the obtained peptide products.

The plasmid contains a regulatory sequence operatively linked to a DNA encoding a desired multi-modular biosynthetic enzyme such as a PKS. Expression systems suitable for use in the present invention include systems that function in eukaryotic host cells and prokaryotic host cells. However, a prokaryotic system is preferable as described above, and a system compatible with bacteria belonging to the genus Streptomyces is particularly important. The regulatory sequences to be used in such a system include a promoter, a ribosome binding site, a terminator, an enhancer, and the like. A useful promoter is a promoter that functions in host cells of the genus Streptomyces, and examples thereof include pGapdh, pErmE, pKasO, and the like, but the examples are not limited thereto.

The plasmid can also contain a selectable marker. Various markers are known, including a gene that is useful in the selection of a transformed cell line and generally imparts a selectable phenotype to the transformed cells upon expression when the cells are grown in a suitable selection medium. Such markers include, for example, a gene that imparts antibiotic resistance or sensitivity to the plasmids. Alternatively, some polyketides are naturally colored, and this characteristic provides a built-in marker for selecting cells that have been successfully transformed with the construct of the present invention.

A method for introducing the plasmid of the present invention into a suitable host is known to those skilled in the art, and the method typically includes the use of CaCl₂ or a divalent cation and other additional agents such as DMSO. A DNA can also be introduced into a bacterial cell by electroporation. Once a PKS, which is a multi-modular biosynthetic enzyme, is expressed, polyketide-producing colonies can be identified and isolated using a known technique. The plasmid of the present invention may be introduced into a host cell using conjugal transfer between bacteria. In one preferred embodiment of the present invention, a base region encoding a PKS is transferred to a plasmid of Escherichia coli, and is transferred from Escherichia coli to an actinomycete by conjugation. The DNA encoding a PKS is incorporated in genome of a host cell such as an actinomycete. In a case where the host cell is an actinomycete, the genus Streptomyces is preferable.

The plasmid containing a plurality of genes encoding a multi-modular biosynthetic enzyme of the present invention contains a DNA encoding a domain contained in the multi-modular biosynthetic enzyme, and the type and size thereof are not particularly limited. The type of the DNA encoding a domain contained in the multi-modular biosynthetic enzyme is not particularly limited, and may be a DNA containing not only a sequence naturally derived from a microorganism or the like, but also an artificially designed sequence. Preferred examples of the gene cluster include a gene cluster constituting a PKS or NRPS. In the naturally-derived DNA sequence, one codon is usually used in a predetermined origin organism by the organism to express the corresponding amino acid. However, in the case of heterologous expression, it is required to be matched with the codon usage frequency of the host. Examples of other factors that may influence the result of the heterologous expression include a GC content (a content of base guanine and cytosine in the sequence), and a repetitive sequence. The repetitive sequences lower the genetic stability, generate a risk of incorrect hybridization, and inhibit the synthesis of repetitive segments. Therefore, the synthetic gene needs to be optimized in relation to the codon usage and GC content. However, it is generally difficult to optimally satisfy these conditions at the same time. For example, optimization of the codons can result in an unusually repetitive DNA sequence or high GC content. In the present invention, the GC content is 30 to 70%. The GC content is preferably 70% or less, 68% or less, 65% or less, or 60% or less. By using the present invention, it is possible to synthesize a target plasmid with high efficiency even when the GC content is 50% or more, 52% or more, 55% or more, 58% or more, or 60% or more. Preferably, the codon is optimized to prevent repetition of base sequences of 20 bp or more from appearing. It is preferable to avoid an extreme difference in GC content in the gene. For example, it is preferable that the difference between the highest and lowest GC contents within a 50 bp stretch is 52% or less. The amount of homopolymers is preferably reduced as much as possible. It is preferable to minimize the number/length of small repeats dispersed within the DNA sequence as much as possible.

### [DNA assembly method using Bacillus subtilis]

In the present specification, the DNA assembly method using Bacillus subtilis is not particularly limited as long as it is a DNA assembly method using Bacillus subtilis, and examples thereof include the methods described in JP 2004-129654 A, JP 2005-253462 A, and JP 2007-135533 A.

### (OGAB method)

In the present specification, an ordered gene assembly in Bacillus subtilis method (OGAB) is a multiple DNA fragment assembly method using a plasmid transformation system of Bacillus subtilis. Specifically, the method is a method in which a DNA fragment to be assembled and an assembled plasmid vector are prepared so as to have a specific protrusion of 3 and 4 bases, and the DNA fragments to be linked are linked by specifying the order and orientation of the DNA fragments using this complementarity.

In addition, examples thereof include the methods described in JP 2004-129654 A, which are methods for easily obtaining a plasmid DNA in which a plurality of DNA fragments are linked and assembled in a certain order and orientation and which may be amplified in a microorganism by using a DNA uptake capability and homologous recombination capability of a microorganism such as Bacillus bacteria, and a method for obtaining a microorganism in which a DNA sequence in which a plurality of DNA fragments are linked and assembled in a certain order and orientation is contained in genomic DNA.

By utilizing the fact that the protruding ends of three bases generated by digestion of a restriction enzyme SfiI of a DNA can be specified to an arbitrary sequence, SfiI cleavage sites are designed and prepared by producing ends at which a DNA fragment of a constituent element to be assembled and a linear plasmid vector fragment having an effective replication mechanism in Bacillus subtilis, so that the respective fragments can be sequentially linked once in one DNA assembly unit, these SfiI fragments are mixed at the same concentration so as to be equimolar, and then a ligation reaction is performed in the presence of polyethylene glycol and a salt, such that a linear polymeric DNA having a structure in which the DNA linking unit is generated, and is transformed into Bacillus subtilis competent cells, and therefore, it is possible to link the DNAs in a desired order and orientation into a Bacillus subtilis plasmid. In addition, a Bacillus subtilis competent cell in which a sequence common to the sequence in the plasmid is inserted into genomic DNA and a linear polymeric DNA having a structure in which the DNA linking unit obtained above is multiply repeated are co-cultured, such that DNAs can be linked in the Bacillus subtilis genomic DNA in a desired order and orientation.

### (Combi-OGAB method)

A combi-OGAB method is the method described in WO 2020/203496 A, and is a method in which in a gene assembly method (OGAB method) using a plasmid transformation system of Bacillus subtilis, a ratio of molar concentrations of all DNA fragments used for assembly of a combinatorial library is set as close to 1 as possible. Specifically, a species plasmid is constructed by linking all the alternative gene fragments to be combinatorialized. Also, for other alternative gene fragments, species plasmids are separately constructed to prepare the number of species plasmids equal to the maximum number of alternatives. By cleaving various plasmids with a restriction enzyme, a solution in which gene fragments are once mixed in equimolar amounts is obtained. The solution maintains equimolar properties when mixed with other species plasmids. Thereafter, various gene fragments contained in the solution are linearly linked to obtain a polymeric DNA in a pseudo tandem repeat state in which a plasmid vector moiety periodically appears, and Bacillus subtilis is transformed using the polymeric DNA. A combinatorial library is efficiently constructed by circularization utilizing the homology of plasmid vector moieties within Bacillus subtilis.

According to this method, it is possible to extremely easily and reliably prepare equimolar gene fragments required for construction of a combinatorial library, and a construction scale of the library can be set to be unconventionally large.

### [First gene cluster]

The first gene cluster of the present invention is a gene cluster containing a plurality of genes encoding a multi-modular biosynthetic enzyme.

### [Second gene cluster]

The second gene cluster of the present invention is a gene cluster containing a replication origin of Bacillus subtilis, a replication origin of Escherichia coli, and an initiation sequence for conjugation to an actinomycete.

### [Replication origin of Bacillus subtilis]

The replication origin of Bacillus subtilis of the present invention is not particularly limited as long as it can exhibit its function.

### [Replication origin of Escherichia coli]

The replication origin of Escherichia coli of the present invention is not particularly limited as long as it can exhibit its function, and examples thereof include RepA.

### [Initiation sequence for conjugation to actinomycete]

The initiation sequence for conjugation to an actinomycete of the present invention is not particularly limited as long as it can exhibit its function.

### [Plasmid]

The plasmid of the present invention contains a replication origin of Bacillus subtilis, a replication origin of Escherichia coli, and an initiation sequence for conjugation to an actinomycete, and may contain a prokaryotic F factor distribution system for single copy maintenance in Escherichia coli, a site-specific recombination system that enables integration of a vector into a genome of a recipient host at a defined location, one or a plurality of selectable markers that function in Bacillus subtilis, Escherichia coli, and an actinomycete expression host, and the like.

The replication origin of Bacillus subtilis is not particularly limited, examples thereof include those set forth in SEQ ID NO: 3, and the homology with SEQ ID NO: 3 may be 80% or more, 85% or more, 90% or more, 93% or more, 95% or more, 97% or more, or 99% or more.

The replication origin of Escherichia coli is not particularly limited, examples thereof include those set forth in SEQ ID NO: 4 in the replication origin of Escherichia coli, and the homology with SEQ ID NO: 4 may be 80% or more, 85% or more, 90% or more, 93% or more, 95% or more, 97% or more, or 99% or more. The initiation sequence for conjugation to an actinomycete is not particularly limited, examples thereof include those set forth in SEQ ID NO: 6, and the homology with SEQ ID NO: 6 may be 80% or more, 85% or more, 90% or more, 93% or more, 95% or more, 97% or more, or 99% or more.

The prokaryotic F factor distribution system for single copy maintenance in Escherichia coli is not particularly limited, examples thereof include those set forth in SEQ ID NO: 5, and the homology with SEQ ID NO: 5 may be 80% or more, 85% or more, 90% or more, 93% or more, 95% or more, 97% or more, or 99% or more.

The site-specific recombination system that enables integration of a vector into a genome of a recipient host at a defined location is not particularly limited, examples thereof include those set forth in SEQ ID NO: 7, and the homology with SEQ ID NO: 7 may be 80% or more, 85% or more, 90% or more, 93% or more, 95% or more, 97% or more, or 99% or more.

The one or the plurality of selectable markers that function in Bacillus subtilis, Escherichia coli, and an actinomycete expression host are not particularly limited, examples thereof include those set forth in SEQ ID NO: 8, and the homology with SEQ ID NO: 8 may be 80% or more, 85% or more, 90% or more, 93% or more, 95% or more, 97% or more, or 99% or more.

In one embodiment, an OGAB vector 1.0 (SEQ ID NO: 22), an OGAB vector 2.0 (SEQ ID NO: 23), an OGAB vector 2.1 (SEQ ID NO: 24), and an OGAB vector 2.2 (SEQ ID NO: 25) are exemplified, and the homology with SEQ ID NO: 22, SEQ ID NO: 23, SEQ ID NO: 24, and SEQ ID NO: 25 may be 80% or more, 85% or more, 90% or more, 93% or more, 95% or more, 97% or more, or 99% or more.

### [Method for producing multi-modular biosynthetic enzyme]

A method for producing a multi-modular biosynthetic enzyme of the present invention is a method for producing a plasmid containing a plurality of genes encoding a multi-modular biosynthetic enzyme, and it is possible to use a plasmid produced by the method for producing a plasmid, the method including: preparing a first gene cluster containing the plurality of genes by a DNA assembly method using Bacillus subtilis; and linking, to the first gene cluster, a second gene cluster containing a replication origin of Bacillus subtilis, a replication origin of Escherichia coli, and an initiation sequence for conjugation to an actinomycete.

In addition, it is possible to use a plasmid produced by an OGAB method as the DNA assembly method using Bacillus subtilis.

The multi-modular biosynthetic enzyme can be performed by a known method for producing a multi-modular biosynthetic enzyme in a host cell using a plasmid, and in one embodiment, a transformant in which a plasmid is introduced into a host cell is cultured, and a multi-modular biosynthetic enzyme can be obtained from the culture. The "culture" refers to any of the culture supernatant, cultured cells, cultured bacterial cells, or disrupted cells or bacterial cells. A method for culturing the transformant of the present invention can be performed in accordance with an ordinary method used for host culturing.

Any of a natural medium or a synthetic medium may be used as a medium for culturing the transformant of the present invention, as long as the medium contains a carbon source, a nitrogen source, inorganic salts, and the like that the host can assimilate, and thus the culturing of the transformant can be performed efficiently. Examples of the carbon source include a carbohydrate such as glucose, galactose, fructose, sucrose, raffinose, and starch, an organic acid such as acetic acid and propionic acid, and alcohols such as ethanol and propanol. Examples of the nitrogen source include ammonia, an ammonium salt of an inorganic acid or an organic acid, such as ammonium chloride, ammonium sulfate, ammonium acetate, or ammonium phosphate, and other nitrogen-containing compounds. In addition, peptone, meat extract, corn steep liquor, various amino acids, or the like may be used. Examples of the inorganic substance include monopotassium phosphate, dipotassium phosphate, magnesium phosphate, magnesium sulfate, sodium chloride, ferrous sulfate, manganese sulfate, copper sulfate, and calcium carbonate.

The culturing is generally performed under an aerobic condition such as shaking culture or aeration and agitation culture at 28 to 38°C. pH adjustment is performed using an inorganic or organic acid, an alkaline solution, or the like.

When the culture is performed under the above culture conditions, a multi-modular biosynthetic enzyme can be produced with a high yield.

After the culture, when the multi-modular biosynthetic enzyme is produced in bacteria or cells, the expression product can be collected by subjecting the bacteria or cells to homogenizer treatment or the like to disrupt the bacteria or cells. On the other hand, when polyketides are transported outside bacteria or outside cells, the culture solution is used as it is, or the bacteria or cells are removed by centrifugation or the like. Thereafter, the expression product is collected from the culture by extraction by ammonium sulfate precipitation or the like, and further isolated and purified using various types of chromatography or the like as necessary.

The host cell for producing the multi-modular biosynthetic enzyme is not particularly limited, and it is preferable to use an actinomycete. When the multi-modular biosynthetic enzyme is a PKS, as the host cell, an actinomycete is preferable used, and the genus Streptomyces is more preferable. The greatest merit of using the host cell of the genus Streptomyces is that a production titer is higher than that in heterologous expression production using Escherichia coli, and there is a post-translational modification system that is essential for the activity expression of the type I PKS. Specific examples of the host cells of the genus Streptomyces include S. albus, S. ambofaciens, S. avermitilis, S. azureus, S. cinnamonensis, S. coelicolor, S. curacoi, S. erythraeus, S. fradiae, S. galilaeus, S. glaucescens, S. hygroscopicus, S. lividans, S. parvulus, S. peucetius, S. rimosus, S. roseofulvus, S. thermotolerans, and S. violaceoruber, and S. albus is preferable.

A method for introducing a plasmid containing a plurality of genes encoding a multi-modular biosynthetic enzyme into an actinomycete can be performed by a known method, and is not particularly limited, and examples thereof include a method for introducing a plasmid into an actinomycete from Escherichia coli by conjugation.

In a case where the plasmid containing a plurality of genes encoding a multi-modular biosynthetic enzyme is further a plasmid containing a replication origin of Bacillus subtilis, a replication origin of Escherichia coli, and an initiation sequence for conjugation to an actinomycete, the host cell for producing the multi-modular biosynthetic enzyme is designated as an actinomycete, such that there is an advantage that the multi-modular biosynthetic enzyme can be produced without modifying the plasmid prepared by the DNA assembly method using Bacillus subtilis to a structure suitable for Escherichia coli and an actinomycete, and the steps required for producing the multi-modular biosynthetic enzyme can be reduced. In addition, it is not required to modify the plasmid for each host cell to be transformed, and it is possible to reduce the risk of mutation such as insertion or deletion occurring when the plasmid is amplified. The produced multi-modular biosynthetic enzymes can be usefully used in various fields.

### Examples

Hereinafter, the present invention will be described based on Examples, but the present invention is not limited to these Examples.

### Example 1

### <DNA assembly using Bacillus subtilis>

Some DNA sequence information on Kitasatospora aureofaciens was obtained from the MiBig Repository of Known Biosynthetic Gene Clusters (https://mibig.secondarymetabolites.org/repository/BGC00010 03/index.html#r1c1), redesign such as codon optimization was performed, synthesis with all DNA fragments (SEQ ID NOs: 9 to 21) of a target PKS cluster construct was performed by Twist Bioscience, amplification was performed with Escherichia coli, and re-extraction was performed.
GC content before codon optimization (%):
   LipPKS1: 74.2%
   LipPKS2: 72.3%
   LipPKS3: 71.3%
   LipPKS4: 71.3%
   LipNRPS: 75.2%
   LipMT: 71.1%
   LipTE: 74.5%
GC content after codon optimization (%):
   LipPKS1: 63.7%
   LipPKS2: 63.7%
   LipPKS3: 63.9%
   LipPKS4: 63.9%
   LipNRPS: 65.3%
   LipMT: 59.6%
   LipTE: 65.6%

A concentration of all the DNA fragments (SEQ ID NOs: 9 to 21) was measured with a UV spectrophotometer (Thermofisher Nanodrop) and adjusted to produce equimolar fragment mixtures.

50 µl of the DNA fragments, 2 µl of DNase (Lucigen Corporation (Plasmid-Safe)), 2.4 µl of 15mM ATP, and 6 µl of 10 × Buffer were mixed, and the concentration of each DNA fragment was adjusted to 100 ng/µl.

500 ng of each DNA fragment was collected in a tube and mixed with 5% BsmbI-HFv2 (NEB) in terms of total reaction amount.

In order to purify the treated DNA fragments, phenol-chloroform treatment, butanol treatment, and ethanol precipitation were performed.

Target fragments were removed from the treated plasmid mixture by performing gel extraction using a dialysis tube, and the treated target fragments were precipitated and purified by ethanol precipitation.

In order to obtain a DNA construct containing tandem repeats of a DNA fragment encoding a target PKS cluster, the treated DNA fragments were mixed with OGAB vector 2.0 (SEQ ID NO: 23), 1 µl of T4 DNA ligase (TAKARA BIO), and a ligation buffer, and the mixture was incubated at 37°C for 3 hours to complete the ligation.

The DNA ligation solution containing the DNA construct was mixed with Bacillus subtilis competent cells, and the cells were mixed at 37°C for 90 minutes. After the incubation period, the cells were inoculated on a tetracycline selective plate.

After a period of colony growth, the transformants were picked up from the plate and cultured overnight in 2 ml of LB medium at 37°C.

Plasmid extraction was performed according to a known procedure, and it was confirmed whether the obtained DNA was assembled as expected.

### Example 2

### <Introduction of plasmid from Escherichia coli to actinomycete>

A normally assembled OGAB shuttle vector construct containing the target PKS cluster was transformed into Escherichia coli ET12567/pUZ8002 competent cells and cultured on a plate containing apramycin and selected.

Transformants were selected and cultured overnight in 10 ml of LB medium containing chloramphenicol, kanamycin, and apramycin.

The culture was diluted in fresh LB medium and antibiotics (chloramphenicol, kanamycin, and apramycin) at 1:100 and cultured so that OD600 was 0.4〜0.6.

Escherichia coli cells were washed twice with an equal volume of LB medium to remove any antibiotics that might inhibit actinomycetes and re-suspended in 0.1 volumes of LB medium.

Plates for S. albus J1074 spores were prepared individually and extraction was performed with water to prepare a spore solution of actinomycetes. 500 µL of YT medium was added to 500 µl of the spore solution. The obtained spore solution mixture was subjected to heat shock at 50°C for 10 minutes and allowed to cool.

500 uL of Escherichia coli cells were added to 500 uL of the heat-shocked spore solution. The obtained mixture was centrifuged in a centrifuge at 11,000 rpm for 1 minute. A supernatant was left at about 100 µl, and the pellets were re-suspended in a residual liquid with the residue.

The obtained mixture was plated on MS agar plate + 10 mM MgCl, and incubation was performed at 30°C for 16〜20 hours.

After the incubation, the plate was covered with 1 ml of water containing 0.5 nalidixic acid and 1 mg of apramycin, and incubation was further continued at 30°C for about 4 days.

Potential conjugates were confirmed in selective media containing nalidixic acid.

## Claims

1. A method for producing a plasmid containing a plurality of genes encoding a multi-modular biosynthetic enzyme, the method comprising: preparing a first gene cluster containing the plurality of genes by a DNA assembly method using Bacillus subtilis; and linking, to the first gene cluster, a second gene cluster containing a replication origin of Bacillus subtilis, a replication origin of Escherichia coli, and an initiation sequence for conjugation to an actinomycete.

2. The method for producing a plasmid according to claim 1, wherein the DNA assembly method using Bacillus subtilis is an OGAB method.

3. A plasmid produced by the method according to claim 1 or 2.

4. An actinomycete comprising a plasmid produced by the method according to claim 1 or 2.

5. A method for producing a multi-modular biosynthetic enzyme, the method comprising producing a multi-modular biosynthetic enzyme in a host cell containing a plasmid produced by the method according to claim 1 or 2.

6. The method for producing a multi-modular biosynthetic enzyme according to claim 5, wherein the host cell is an actinomycete.

7. The method for producing a multi-modular biosynthetic enzyme according to claim 6, wherein the actinomycete is an actinomycete containing a plasmid obtained by conjugal transfer with Escherichia coli.

8. The method for producing a multi-modular biosynthetic enzyme according to any one of claims 5 to 7, wherein the multi-modular biosynthetic enzyme is a type I polyketide synthase (PKS).

9. A multi-modular biosynthetic enzyme produced by the method according to any one of claims 5 to 8.

10. A plasmid comprising at least all of the following:
(a) a replication origin of Bacillus subtilis; and
(b) a replication origin of Escherichia coli.

11. A DNA encoding a PKS having a homology of 80% or more with a DNA sequence encoding a PKS set forth in SEQ ID NO: 2.

12. A plasmid having a homology of 80% or more with a DNA sequence encoding a plasmid set forth in SEQ ID NO: 23.
